# EUROPEAN PATENT SPECIFICATION

(11) **EP 0 110 385 B1**
(45) Date of publication and mention of the grant of the patent: **17.02.1993**
(21) Application number: 83111976.3
(22) Date of filing: 29.11.1983
(51) Int. Cl.: C12N 15/86, C12N 9/10, A61K 39/12, A61K 39/00, C12N 15/39, C07K 15/00

(54) **Process for producing poxvirus recombinants for expression of foreign genes**
Verfahren zur Herstellung von Poxvirus-Rekombinanten zur Expression fremder Gene
Procédé de préparation de recombinants de poxvirus pour l'expression de gènes étrangers

(30) Priority: 30.11.1982 US 445451; 01.12.1982 US 445892
(43) Date of publication of application: 13.06.1984
(73) Proprietor: THE UNITED STATES OF AMERICA as represented by the Secretary United States Department of Commerce, Springfield, Virginia 22161 (US)
(72) Inventor: Moss, Bernard, Bethesda Maryland 20852 (US); Smith, Geoffrey Lilley, Rockville Maryland (US); Mackett, Michael, Bethesda Maryland 20814 (US)
(74) Representative: Perry, Robert Edward

(56) References cited:
- EP-A- 0 074 808
- EP-A- 0 083 286
- Proc. Natl. Acad. Sci. USA, Vol. 80, September 1983, no. 17, Genetics, Washington, US, D. PANICALI et al.: "Construction of live vaccines by using genetically engineered poxviruses: Biological activity of recombinant vaccina virus expressing influenza virus hemagglutinin" pages 5364-5368
- NATURE, vol. 302, April 1983, no. 5908, Chesham, Bucks, GB, G.L. SMITH et al.: "Infectious vaccinia virus recombinants that express hepatitis B virus surface antigen", pages 490-495
- CHEMICAL ABSTRACTS, vol. 99, no. 9, August 29, 1983, page 179, ref. 65367p, Columbus, Ohio, US & US- A-445 892 (UNITED STATES DEPT. OF HEALTH AND HUMAN SERVICES)
- CHEMICAL ABSTRACTS, vol. 99, no. 19, November 7, 1983, page 167, ref. 153207s, Columbus, Ohio, US & US - A - 445 451 (UNITED STATES DEPT. OF HEALTH AND HUMAN SERVICES)
- Proc. Natl. Acad. Sci, USA vol. 79, Februari 1982, Genetics, J.P. WEIR et al.: Mapping of the vaccinia virus thymidine kinase gene by marker rescue and by cell-free translation of selected mRNA", page 1210-1214
- CHEMICAL ABSTRACTS, vol. 95, no. 21, November 23, 1981, page 228 ref. 182552y, Columbus, Ohio, US, S. VENKATESAN et al.: "Distinctive nucleotide sequences adjacent to multiple initiation and termination sites of an early vaccina virus gene" & Cell (Cambridge Mass), 1981, 25(3), 805-13 (Eng.)
- Proc. Natl. Acad. Sci. USA, vol. 79, August 1982, Biochemistry, D. PANICALI et al.: "Construction of poxviruses as cloning vectors: Insertion of the thymidine kinase gene from herpes simplex virus into the DNA of infectious vaccinia virus", pages 4927-31
- Proc. Natl. Acad. Sci, USA, vol. 79, December 1982, Genetics M. MACKETT et al.: "Vaccinia virus: A selectable eukaryotic cloning and expresion vector", pages 7415-7419

## Description

Recombinant DNA technology has made it possible to express genes of one organism within another. The prior art shows that several virus groups including the papovaviruses, papilloma viruses, adenoviruses, and retroviruses have been employed as eukaryotic molecular cloning and expression vectors. The relatively small sizes of these virus genomes have facilitated the in vitro construction of recombinant DNA molecules. However, they generally exhibit a limited host range, provide severe limitations on the amounts of DNA that can be accommodated and suffer loss of infectivity upon insertion of foreign DNA. Although genetic engineering of larger viruses, such as poxviruses, is more difficult, such vectors could have the advantage of greater capacity and potential of retaining infectivity in a wide range of host cells. For poxviruses such as vaccinia virus, such recombinants may lead to the development of live virus vaccines.

Since vaccinia virus is the best studied member of the poxvirus group, it will be described here. Vaccinia virus has a very broad host range in vitro and in vivo and has been used world-wide as an effective vaccine against variola, a related poxvirus that causes smallpox. Vaccinia is a large virus containing a linear double-stranded DNA genome with a molecular weight of about 122 million, equivalent to more than 180,000 base pairs. The virus uses its own enzymes for transcription and replication within the cytoplasm of infected cells. Nucleotide sequence data indicate that the transcriptional regulatory signals encoded in the vaccinia virus genome are distinct from those used by eukaryotic cells. The invention described here takes into account both the large size of the poxvirus genome and its unique transcriptional regulatory signals.

References which relate to the present invention are Venkatesan et al, Cell 125:805-813 and J. Virol. 44:637-646 (1982); Baiszer et al, J. Virol. 45:62-72 (1983); Weir et al, J. Virol. 46:530 (1983); Moss et al, J. Virol. 40:387-395 (1981); Mackett et al, PNAS USA 79:7415 (1982); US-A- 4237224; Valuenzuela et al, Nature 298:347-350 (1982); Moriarty et al, PNAS USA 78:2606-2610 (1981); and Liu et al, DNA 1:213-221 (1982).

Panicali and Paoletti, PNAS USA 79:4927-4931 (1982), describe donor or insertion vectors for the preparation of recombinant vaccinia virus. The vectors comprise the vaccinia Hind III F fragment into which, at the BamHI site, the thymidine kinase gene from herpes simplex virus (HSV TK) has been inserted (cf. pDP132 and pDP137 in Figure 1). The HSV TK gene contains the gene's own regulatory signals (page 4927, column 1, lines 15-16). The document states that, although results obtained from transcription analysis might be interpreted to mean that the regulatory signals of the HSV TK gene are recognised, "other (unpublished) data suggest that vaccinia signals may be operative for HSV TK expression".

Weir et al, PNAS USA 79:1210-1214 (1982), describe the mapping of the TK gene of vaccinia virus by two independent methods; marker rescue and cell-free translation. Marker rescue established that TK⁺ virus could be selectively titered in the presence of excess "live" TK⁻ virus. Plasmids containing vaccinia virus DNA segments were used to select virus mRNA by a process of hybridisation. Enzymatically-active TK was formed from a cell-free translation of such mRNAs.

### SUMMARY OF THE INVENTION

According to the present invention, a plasmid, cosmid or phage vector which can undergo homologous recombination in a poxvirus, comprises:
a chimeric gene which comprises at least one poxvirus transcriptional regulatory sequence and, under the transcriptional control of the regulatory sequence, at least one uninterrupted protein coding sequence from a foreign gene, wherein the regulatory sequence and the coding sequence are not separated by another transcriptional regulatory sequence; and
DNA, flanking the chimeric gene, from a non-essential region of poxvirus genome.

When the foreign gene is an appropriate one from a pathogen, the recombinant poxvirus can serve as a live vaccine. Some examples of such foreign genes include DNA genes or DNA copies of RNA genes from hepatitis B virus, hepatitis A virus, hepatitis non-A, non-B virus, influenza virus, herpesvirus, cytomegalovirus, adenoviruses, parvoviruses, foot and mouth disease virus, poliovirus, measles virus, rabies virus, coronaviruses, coxsackieviruses and pathogenic bacteria, rickettsia, protazoa, and metazoa. In accordance with the present invention, cells infected with poxvirus recombinants are also used to prepare the foreign gene product.

In considering the development of vaccinia virus or other poxviruses as infectious expression vectors, the following biological characteristics of these agents were taken into account: evidence that vaccinia virus has evolved its own transcriptional regulatory sequences; its large genome size; and lack of infectivity of isolated viral DNA.

With the present invention, efficient expression of foreign DNA is obtained by forming chimeric genes consisting of a poxvirus transcriptional regulatory sequence and an uninterrupted protein coding sequence of a foreign gene. The poxvirus transcriptional regulatory sequence consists of a DNA segment that precedes and may include the site at which RNA synthesis begins. In the description which follows, sequences that positively regulate the transcription of a gene may be referred to as a "promoter." The foreign gene protein coding sequence includes the site corresponding to initiation of translation and will be referred to hereinafter as the "foreign gene." By using the translational initiation site of the foreign gene in accordance with the present invention, codon phasing and potential problems associated with the biological activity of fusion proteins are avoided. The chimeric gene is flanked by DNA from a known non-essential region of the poxvirus genome that will ultimately allow homologous recombination to occur. The present invention thus provides a general method of expressing foreign genes, in that plasmids can be constructed that contain multiple restriction endonuclease sites next to the poxvirus promoter and that contain the flanking poxvirus DNA as well as the plasmid origin of replication and antibiotic resistance gene. The plasmids are then cleaved with an appropriate restriction endonuclease to form ligatable termini, and a foreign gene with complementary termini is ligated next to the poxvirus promoter. The plasmid containing the chimeric gene and flanking poxvirus DNA is used to transform bacteria and then is purified from the transformed bacteria.

The plasmid containing the chimeric gene flanked by poxvirus DNA is then used in accordance with the present invention under transfecting conditions to transfect cells that have been infected with vaccinia virus or another compatible poxvirus. Homologous recombination and replication are allowed to occur, whereupon the chimeric gene is inserted into the poxvirus genome at the position specified by the flanking DNA used. It is important to use flanking DNA from a non-essential region of the genome so that infectivity will not be destroyed.

Recombinant poxvirus provided by the present invention is distinguishable from the original virus by a variety of methods. For example, by using segments of the vaccinia virus thymidine kinase (TK) gene as flanking DNA, the chimeric gene can be inserted into the thymidine kinase gene within the vaccinia virus and thereby interrupt its function. Thus, if the flanking DNA is from the TK gene of vaccinia virus, then virus recombinants lacking TK activity can be selected. Alternatively, virus can be distinguished either by DNA·DNA hybridization, by detection of the product of the foreign gene, or by other selective procedures.

### DETAILED DESCRIPTION OF PREFERRED EMBODIMENTS

The following description of this invention will be divided into the following sections:
I. Preparation of plasmid vector containing poxvirus promoter, sites for insertion of a foreign gene, and poxvirus DNA flanking sequences.
II. Preparation and insertion of foreign gene into the plasmid vector to form chimeric gene.
III. Transfection of virus-infected cells with plasmid containing chimeric gene.
IV. Isolation of recombinant poxvirus and detection of foreign gene product.
V. Infection of susceptible cells or animals with vaccinia virus recombinants.

Throughout the following description, manipulations of vaccinia virus DNA, such as, for example, the locating of the thymidine kinase gene in the vaccinia viral genome, are detailed as exemplary of those readily applicable to poxvirus generally. Accordingly, it is understood that the examples presented herein are illustrative only, and that various changes and modifications within the scope of the present invention will be apparent to those skilled in the art.

### I. Preparation of plasmid vector containing vaccinia virus promoter, sites for insertion of a foreign gene, and vaccinia virus DNA flanking sequences.

The vehicle used to assemble the insertion vector may be any convenient plasmid, cosmid, or phage. Examples of phages which can be used in accordance with the invention are the lambda and M13 phages. An exemplary cosmid which can be used is designated pHC79 and is described by Hohn and Collins, Gene 11:291 (1980). Examples of plasmids which can be used are pBR322, pBR325, pBR327, pBR328, pUC7, pUC8, or pUC9. The vaccinia virus DNA segment used to promote transcription of the foreign gene in the present example contained nucleotide sequences preceding and including the start site of an RNA. The nucleotide sequence and a precise transcriptional map of this region was needed for application of this method. When a convenient restriction endonuclease site preceded the RNA start site and another occurred after the RNA start site but before the first ATG or translational initiation codon, the promoter segment was excised by restriction endonuclease digestion and isolated by standard methods such as agarose gel electrophoresis. When a convenient restriction endonuclease site was not available, it was necessary to use other methods such as cleaving beyond the desired site and removing extra nucleotides with an exonuclease such as Bal31. See MANIATIS et al, MOLECULAR CLONING: A LABORATORY MANUAL (Cold Spring Harbor Laboratory 1982) for a description of such methods and other techniques applied herein.

Either directly or after modification of its ends, the promoter segment was ligated to a plasmid that had been cleaved with a restriction endonuclease to provide compatible ligatible termini. Ligation of cohesive or blunt ends followed standard procedures. Additional restriction endonuclease sites were placed next to the promoter by inserting the promoter into a plasmid such as pUC9 that already has multiple insertion sites; however, ligation of synthetic polynucleotides is also possible. The plasmid containing the promoter was used to transform bacteria and then was purified. Restriction endonucleases were used to excise the promoter with adjacent restriction endonuclease sites, and the resultant DNA fragment was purified using conventional methods.

The DNA used to flank the promoter and added restriction endonuclease sites was derived from a non-essential region of the vaccinia virus genome. Examples of such non-essential regions include the thymidine kinase gene [Weir, Bajszar and Moss, supra; Weir and Moss, supra] and a region of at least 9,000 base-pairs (bp) that is proximal to the left inverted terminal repetition [Moss, Winters and Cooper, supra]. DNA containing the non-essential region was excised by restriction endonuclease cleavage and purified by agarose gel electrophoresis or other conventional methods. The segment was then ligated to plasmid DNA that had been cleaved by a restriction endonuclease to give complementary ligatable termini. The plasmid containing the vaccinia virus DNA was used to transform bacteria and was then purified. An appropriate restriction endonuclease was used to cleave the non-essential segment of the vaccinia virus DNA within the plasmid so that it could be ligated to the previously isolated promoter fragment. By this procedure (or by obvious variations thereof in which the order of ligation and cleavage steps is modified), a plasmid was obtained that has a vaccinia virus promoter with adjacent restriction endonuclease sites flanked by a non-essential segment of vaccinia virus DNA. Since this plasmid retained the plasmid origin of replication and antibiotic resistance gene, it was used to transform bacteria and was replicated.

### II. Preparation and insertion of foreign gene into the plasmid vector to form chimeric gene.

A segment of DNA containing a foreign gene or a cDNA copy of a foreign gene was obtained. The DNA segment was cleaved with restriction endonucleases at a site preceding the translational initiation codon and distal to the end of the protein coding sequences. When appropriate sites were not present, then it was necessary to cleave beyond the desired site and use an exonuclease such as Bal31 to remove extra nucleotides. For optimal expression, the first ATG in the segment was used to initiate translation of the desired gene. Since there is no evidence for splicing of vaccinia virus RNAs, continuous protein coding sequences were used.

The plasmid constructed in part I of this section was cleaved at a restriction endonuclease site next to the promoter. The protein coding segment of the foreign gene was ligated directly to the promoter, when it had complementary termini, or after modification of its ends. The plasmid was used to transform bacteria and then was purified. When the foreign gene was insertable in more than one orientation, it was necessary to analyze by restriction endonuclease digestion and gel electrophoresis or nucleotide sequencing to check that the proper one was obtained. The desired plasmid had the promoter adjacent to the start of the foreign gene.

### III. Transfection of virus-infected cells with plasmid containing chimeric gene.

Plasmids containing chimeric genes flanked by DNA from non-essential regions of the vaccinia virus genome were used to transfect cells that were already infected with vaccinia virus. The chimeric gene was inserted into the vaccinia virus genome by homologous recombination. Typically, confluent monolayers of CV-1, BSC-1, TK 143, or other cells in bottles with a 25 cm² bottom surface area were infected with 0.01 to 0.05 plaque forming units (pfu) per cell of vaccinia virus. Approximately 1 µg of plasmid DNA with or without 1 µg of vaccinia virus DNA and 20 µg of calf thymus DNA or other carrier DNA was mixed in 1 ml of 0.1% dextrose, 0.14 M NaCl, 5 mM KCl, 1 mM Na₂HPO₄, 20 mM Hepes, (pH 7.05) and precipitated by addition of CaCl₂ to a final concentration of 125 mM. The mixture was agitated gently and allowed to remain at room temperature for about 45 min. Two hr after infection, 0.8 ml of the fine suspension was added to an infected monolayer from which medium had been removed. After 30 min, 8 ml of Eagle or other tissue culture medium containing 8% fetal bovine serum was added to each bottle and the incubation was continued at 37°C for 3.5 more hr. At 6 hr after infection, fresh medium containing 8% fetal bovine serum was added and incubation was continued for 48 hr. At this time, the infected cells were scraped off the bottle, centrifuged, resuspended in tissue culture medium and homogenized to break the cells and liberate virus.

### IV. Isolation of recombinant vaccinia virus and detection of foreign gene product.

Virus from transfected cells consisted of a population of which only a small percentage were recombinants. A variety of selective and non-selective methods were used to isolate these recombinants.

Selective procedures depended on the ability of recombinants to replicate under conditions that inhibited the original virus. One selective method involved the inactivation of the vaccinia virus TK gene. This was achieved by using DNA from the vaccinia virus TK gene to flank the chimeric gene. When homologous recombination occurred, the chimeric gene was inserted into the TK gene of virion DNA and the recombinants exhibited a TK negative (TK⁻) phenotype. Selective conditions for isolation of TK⁻ vaccinia virus was achieved by plaguing the virus in monolayers of TK⁻ negative cells such as TK⁻143 cells with 25 µg/ml of 5-bromodeoxyuridine (BUdR) in the 1% low melting agar overlay. After 48 to 72 hr at 37°C in a 5% Co₂ humidified atmosphere, plaques were detected by staining with 0.005% neutral red. Typically, more than 30% of the TK⁻ plaques consisted of recombinants and the remainder were spontaneous TK⁻ mutants of vaccinia virus.

A second selective method was used when TK⁻ cells were infected with TK⁻ mutants of vaccinia virus and then transfected with plasmids that contained a chimeric herpes virus TK gene. [The TK⁻ mutants of vaccinia virus were obtained by infecting TK⁻143 cells with vaccinia virus in the presence of 25 g/ml of BUdR. The TK⁻ negative mutants were then plaqued at least 2 times in succession in TK⁻143 cells in the presence of BUdR.] Recombinants expressing herpesvirus TK were selected by plaque assay on TK⁻143 cells with a 1% low melting agar overlay containing Eagle medium and 8% fetal bovine serum, 100 µM thymidine, 50 µM adenosine, 50 µM guanosine, 10 µM glycine, 1 µM methotrexate. After 48 to 72 hr at 37°C in a 5% CO₂ humidified atmosphere, the plaques were detected by staining with neutral red.

Non-selective methods that depend on identification of virus plaques that contain the foreign gene were also used. In addition, such methods were used to confirm the identity of recombinants even after isolation by selective methods.

DNA·DNA hybridization was used to identify plaques formed by recombinant virus. One method was referred to as dot blot hybridization. In this procedure, virus obtained following transfection of infected cells with chimeric plasmids was plaqued on cell monolayers with a 1% agar overlay. After 48 to 72 hr, the plaques were detected by staining with neutral red. Virus within individual plaques were picked using a sterile Pasteur pipette and used to infect cell monolayers in 16 mm diameter wells of microtiter dishes. After 48 hr incubation at 37°C, the cells were scraped, lysed by three freeze-thaw cycles, and collected on nitrocellulose sheets by filtration using a micro-sample manifold (Schleicher and Schuell, NH). The filter was washed with 100 mM NaCl, 50 mM Tris-HCl (pH 7.5), blotted three times on successive Whatman 3 MM papers saturated with (1) 0.5 M NaOH, (2) 1 M Tris-HCl (pH 7.5), and (3) 2 X SSC (SSC is 0.15 M NaCl, 0.015 M sodium citrate), baked at 80°C for 2 hr and then incubated with 5 X Denhardt's solution [Denhardt, Biochem. Biophys. Res. Commun., 23:641-646 (1966)], supplemented with 0.1 mg/ml of denatured salmon sperm DNA in 4 X SSC at 65°C for 4 hr. The foreign DNA, labeled with ³²P by nick translation, and sodium dodecyl sulfate (SDS) at a final concentration of 0.1% were added and hybridization continued for 12 hr. The filter was washed twice for 15 min at 65°C with 2 X SSC/0.1% SDS and then with 0.2 X SSC/0.1% SDS. An autoradiograph was made by placing the filter next to X-ray film and the presence of dark spots on developed film identified recombinant virus. Another method of DNA·DNA hybridization used was described by Villarreal and Berg [Science 196:183-185 (1977)]. In this method, a replica of virus plaques was made by placing a nitrocellulose filter directly on the cell monolayer. DNA·DNA hybridization was carried out as above and, after location of plaques containing recombinant virus, residual virus was eluted from the agar that originally overlayed the plaques.

Additional methods that depend on expression of the foreign gene were also used to identify plaques. Plaques containing recombinant virus were then identified by autoradiography. When the herpesvirus thymidine kinase was expressed, recombinant plaques were detected by incorporation of [¹²⁵I]deoxycytidine (1 µCi/ml) in the presence of 20 µg/ml of tetrahydrouridine from 14 to 48 hr after infection.

### V. Infection of susceptible cells or animals with vaccinia virus recombinants.

After identification of vaccinia virus recombinants, 2 or more successive plaque purifications were carried out to obtain pure recombinant virus. Susceptible cells such as BSC-1, HeLa, MRC-5, or others were infected to obtain large stocks of recombinant virus. The titers of the stocks were determined by serial dilution and plaque assay.

To express the foreign gene, cells were infected with 1 to 30 pfu/cell of crude or purified virus and incubations were carried out at 37°C for up to 48 hr. The foreign gene product, depending on its nature was found in the cell culture medium or within the cells. When present in the cells, it was liberated by one of a number of methods including sonication, freeze-thawing, homogenization, or detergent treatment. The foreign protein was detected by immunological, enzymatic, and electrophoretic methods.

For infection of animals, recombinant virus was introduced intradermally, although other routes should be satisfactory. Formation of antibodies to the product of the foreign gene indicated that the foreign protein was made and was immunogenic.

### EXAMPLES

In order to demonstrate the subject invention, we made several vectors containing vaccinia virus promoters that can be used for insertion of foreign protein coding sequences to form chimeric genes. Protein coding sequences from other DNA viruses, RNA viruses and prokaryotes were inserted into the plasmids. Vectors containing the chimeric genes then were used to transfect vaccinia virus-infected cells, and the recombinant virus was isolated by selective methods. Expression of the foreign genes was demonstrated in each case. Many of the routine procedures employed are described in detail by MANIATIS et al., supra.

### Example 1 Construction of plasmids pGS20 and pGS21 containing promoter from the 7.5K polypeptide gene (7.5K gene) of vaccinia virus, restriction endonuclease sites for insertion of foreign protein coding sequences, and an interrupted vaccinia virus thymidine kinase gene as flanking DNA.

(a) Isolation of 7.5K promoter DNA. A DNA fragment of approximately 275 bp that precedes and includes the RNA start site of an early vaccinia virus gene coding for a polypeptide known as 7.5K was obtained from a plasmid pAG4 [Venkatesan et al., Cell 125:805-813 (1981)]. 70 µg of pAG4 was digested to completion with 100 units of restriction endonucleases HincII and RSaI (New England Biolabs) in 50 mM NaCl, 10 mM Tris-HCl (pH 7.4), 10 mM MgSO₄, and 1 mM dithiothreitol (DTT) (hereinafter called medium salt restriction buffer) for 2 hr at 37°C. Resulting DNA fragments were separated by electrophoresis for 1 hr at 200 volts through a 1.5% agarose gel containing 40 mM Tris-Acetate (Tris-Ac) (pH 8.0), 20 mM sodium acetate (NaAc), 2 mM EDTA, 18 mM NaCl. The gel was soaked in 1 g/ml ethidium bromide (EtBr) containing agarose gel buffer for 10 min. DNA fragments within the gel were visualized under long wave ultraviolet light and a gel strip containing a 275 bp DNA fragment was excised using a razor blade. DNA within this gel strip was electroblotted onto a sheet of diethylaminoethyl (DEAE)-cellulose in 40 mM Tris-Ac (pH 7.2), 20 mM NaAc, 1 mM EDTA for 45 min at 2.5 mA and eluted from the DEAE-cellulose by shaking in 1.2M NaCl, 40 mM Tris-Ac (pH 7.2), 20 mM NaAc, 1 mM EDTA for 30 min at 25°C. DEAE-cellulose was removed by centrifugation at 12,000 X g for 2 min, and DNA was precipitated from the supernatant by addition of 2 volumes of ethanol and recovered by centrifugation at 12,000 X g for 5 min.
(b) Insertion of 7.5K gene promoter into plasmid pUC9. Two µg of pUC9 DNA was digested with 5 units of restriction endonuclease HincII in medium salt restriction buffer for 2 hr at 37°C. The mixture was heated to 70°C for 5 min and DNA was extracted with an equal volume of phenol:chloroform (1:1) and recovered by ethanol precipitation. DNA was dephosphorylated at its 5' termini by incubation in 50 µl of 50 mM Tris-HCl (ph 9.0), 1 mM MgCl₂, 0.1 mM ZnCl₂, 1 mM spermidine with 0.1 unit of calf-intestinal alkaline phosphatase (Boehringer Mannheim) for 30 min at 37°C to prevent self-ligation in the next step. The mixture was extracted twice with equal volumes of phenol:chloroform and DNA was recovered by ethanol precipitation. 0.5 µg of linearized, dephosphorylated pUC9 DNA was ligated with 0.15 µg of the previously isolated vaccinia promoter DNA in 50 µl of 66 mM Tris-HCl (pH 7.5), 6.6 mM MgCl₂, 10 mM DTT, 0.5 mM ATP together with 1 unit of T₄ DNA ligase at 12°C for 15 hr. 25 µl of ligated DNA mixture was used to transform competent E. coli strain JM103 and 100 µl of transformed cell suspension was mixed with 50 µl of 2% X-gal and 10 µl of 0.1M IPTG and plated onto an L broth plate containing 1.5% bacto-agar (Difco) and 50 µg/ml ampicillin. The plate was incubated at 37°C for 15 hr. White bacterial colonies were picked and grown in 10 ml of L broth containing 50 g/ml ampicillin. Plasmid DNA was purified from 1.5 ml of bacterial cultures by the following procedure (hereafter referred to as minipreparation of plasmid DNA). Bacterial cells were pelleted by centrifugation (12,000 X g, 1 min), resuspended in 0.1 ml of 25 mM Tris-HCl (pH 8.0), 10 mM EDTA, 50 mM glucose, 2 mg/ml lysozyme (lysis solution) and incubated at 4°C for 30 min. 0.2 ml of 0.2 M NaOH, 1% sodium dodecyl sulfate (SDS) was added and the mixture incubated for a further 5 min on ice. 0.15 ml of 3M NaAc (pH 4.8) was added and the mixture incubated on ice for 1 hr followed by centrifugation at 12,000 X g for 5 min. Plasmid DNA present in the supernatant was precipitated by addition of 1 ml ethanol, recovered by centrifugation and redissolved in 0.1 ml of 10 mM Tris-HCl (pH 7.5), 1 mM EDTA (TE buffer). Plasmid DNA preparations were screened for the presence of the vaccinia virus promoter by digestion of 10% of each plasmid preparation with restriction endonucleases HindIII and EcoRI (5 units of each enzyme in medium salt restriction buffer for 1 hr at 37°C). DNA fragments were separated by agarose gel electrophoresis and visualized as described above. Plasmid preparations containing the vaccinia virus promoter were analyzed further to determine the orientation of the vaccinia promoter with respect to plasmid sequences. This was accomplished by digestion with restriction endonucleases HindIII and HindII or HincII and EcoRI (5 units of each enzyme in medium salt restriction buffer at 37°C for 1 hr) followed by agarose gel electrophoresis. A plasmid with the vaccinia promoter reading toward the plasmid's unique BamHI restriction site was called pGS15. This plasmid was purified in large amounts by the following procedure, hereafter called preparation of plasmid DNA. Bacteria containing the required plasmid were seeded into a 400 ml solution of M-9 medium containing 50 µg/ml ampicillin, 150 µg/ml proline, 150 µg/ml leucine, 0.8 µg/ml vitamin B₁ and grown until the optical density at 590 nm reached 0.8. Chloramphenicol was added to a final concentration of 200 µg/ml and the culture was incubated for 12 hr at 37°C. Bacteria were pelleted by centrifugation (5,000 X g, 10 min), washed in 10 mM Tris-HCl (pH 7.5), 0.15 M NaCl, resuspended in 10 ml lysis solution and incubated for 30 min on ice. 20 ml of 0.2 M NaOH, 0.1% SDS were added and the incubation was continued for 5 min on ice, followed by addition of 15 ml of 3M NaAc (pH 4.8) and a further incubation on ice for 1 hr. The mixture was centrifuged (10,000 X g, 10 min). Plasmid DNA was precipitated by addition of 2 volumes of ethanol and recovered by centrifugation at 10,000 X g for 10 min. The DNA pellet was redissolved in 10 ml of TE buffer, the solution extracted twice with equal volumes of phenol:chloroform, the DNA recovered by ethanol precipitation and centrifugation and redissolved in 5 ml of TE buffer. 0.1 mg/ml of ribonuclease (pretreated by boiling for 10 min to inactivate deoxyribonucleases) was added and incubated for 30 min at 37°C. DNA was then precipitated by addition of NaAc (pH 7) to 0.1 M and 1.5 volumes of ethanol and recovered by centrifugation. Remaining RNA was removed from the DNA by dissolving the pellet in 0.3 M NaCl, 10 mM Tris-HCl (pH 7.5), 10 mM EDTA and filtering it through a Sephacryl-S300 column equilibrated in the same buffer. DNA eluting in the first A₂₆₀ nm peak was recovered by ethanol precipitation and centrifugation. DNA was finally dissolved in TE buffer and stored at 4°C.
(c) Changing the HindIII site of pGS15 to an EcoRI site. To enable the insertion of the vaccinia 7.5K gene promoter now cloned in pGS15 into the vaccinia thymidine kinase gene at the unique EcoRI site, it was necessary to change the HindIII site of pGS15 to an EcoRI site. This resulted in the vaccinia promoter and adjacent restriction sites being flanked by EcoRI sites. 20 µg of pGS15 DNA was cleaved with 50 units of HindIII restriction endonuclease in medium salt restriction buffer for 2 hr at 37°C. After extraction with phenol:chloroform, the DNA was recovered by ethanol precipitation and centrifugation. DNA termini were filled in to form blunt-ends by incubation of DNA in 0.2 mM dATP, 0.2 mM dCTP, 0.2 mM dGTP, 0.2 mM dTTP, 0.5 mM DTT, 5 mM MgCl₂, 50 mM Tris-HCl (pH 7.8) with 2 units of DNA polymerase I large fragment (Boehringer Mannheim) at 37°C for 45 min. DNA was recovered after phenol:chloroform extraction by ethanol precipitation and centrifugation. Synthetic EcoRI linkers were phosphorylated at their 5' termini by incubation with 1 unit of polynucleotide kinase in 50 mM ATP, 65 mM Tris-HCl (pH 7.6), 10 mM MgCl₂, 10 mM 2-mercapto-ethanol at 37°C for 30 min. The phosphorylated EcoRI linkers were then ligated onto linearized, blunt-ended pGS15 DNA by incubation at 4°C for 15 hr in 0.5 mM ATP, 66 mM Tris-HCl (pH 7.6), 6.6 mM MgCl₂, 10 mM DTT with one unit of T4 DNA ligase. DNA was then digested for 4 hr with 100 units of EcoRI in high salt restriction buffer [100 mM NaCl, 50 mM Tris-HCl (pH 7.5), 10 mM MgSO₄] and fragments were separated by agarose gel electrophoresis. A 290 bp fragment was purified by electroblotting onto DEAE-cellulose and ligated to pUC9 that had been cleaved with EcoRI and phosphatase treated. Transformation competent E. coli transformed by the ligated plasmid and transformants were selected and grown and the plasmid was amplified and purified as described above. The said plasmid was termed pGS19.
(d) Insertion of 7.5K gene promoter into vaccinia virus thymidine kinase gene. Plasmid pGS8 (derived from pBR328 by insertion of the vaccinia HindIII J fragment containing the vaccinia virus TK gene into the unique plasmid HindIII site deletion of BamHI and EcoRI sites within the plasmid sequences), was grown and purified. 5 µg of pGS8 was digested with EcoRI and recovered after phenol:chloroform extraction and ethanol precipitation. 5' terminal phosphates were removed by treatment with alkaline phosphatase and the DNA was again recovered after phenol:chloroform extraction by ethanol precipitation. 0.5 µg of pGS8 DNA was then ligated together with 0.1 g of the 290 bp DNA fragment containing the vaccinia virus promoter sequence flanked by EcoRI sites. This fragment had been excised from pGS19 by digestion with EcoRI and purified by agarose gel electrophoresis and electroblotting. Ligated DNA was used to transform competent E. coli cells strain HB101 and bacterial clones were screened for a plasmid containing the inserted vaccinia promoter sequence. Two such plasmids were amplified and purified; each contained the vaccinia promoter sequence but in opposite orientations with respect to plasmid sequences. The clones were termed pGS20 and pGS21. Both of these vectors have BamHI and SmaI restriction sites for insertion of foreign genes downstream from the translocated vaccinia 7.5K gene promoter and are flanked by vaccinia DNA sequences encoding segments of the thymidine kinase gene.

### Example 2 Construction of plasmids pMM3 and pMM4 that contain the promoter of the vaccinia virus thymidine kinase gene, restriction endonuclease sites for insertion of foreign protein coding sequences, and flanking DNA including part of the thymidine kinase gene.

(a) Construction of pMM1. The recent mapping and sequencing of the vaccinia virus thymidine kinase (TK) gene (Weir, Baiszar and Moss, supra; Weir and Moss, supra) allowed us to develop a strategy for isolating the TK promoter with its transcriptional initiation site but devoid of its translational start site. Inspection of the sequence showed a GTC between the transcriptional and translational start sites. If this sequence were ligated to GAC, a sequence GTCGAC recognized by several restriction enzymes would be created. This was achieved in the following manner. 25 µg of a plasmid derived from pUC9 by insertion of the vaccinia HindIII J fragment was cleaved with 50 units of ClaI (Boehringer Mannheim) for 2 hr at 37°C in 10 mM Tris-HCl (pH 8), 10 mM NaCl, giving a linear DNA molecule. The buffer composition was altered to 20 mM Tris HCl (pH 8.1), 100 mM NaCl, 12 mM CaCl₂, 1 mM NA₂EDTA and the solution was preincubated at 30°C for 10 min. Two units of the exonuclease Bal31 were added and 6 g samples were removed at 1, 2, 5 and 10 min after addition of the nuclease. 1 µg of each of the samples was digested with 2 units of HindIII (Bethesda Research Labs) in medium salt restriction buffer at 37°C for 2 hr and the resulting fragments separated by electrophoresis on a 1% agarose gel. The time of Bal31 exonulcease digestion that gave an average size for the smallest fragment of 500 bp was chosen for further manipulation. Five µg of the 10 min nuclease digested sample was phenol extracted and ethanol precipitated. The DNA was cleaved with 10 units of HincII at 37°C for 2 hr in medium salt restriction buffer. The buffer composition was altered to 40 M with respect to dATP, dCTP, dGTP, dTTP, 200 mM NaCl, 75 mM Tris-HCl (pH 8.8) and 10 mM MgCl₂ and 1 unit of E. coli DNA polymerase I (large fragment) was added and the reaction incubated at room temperature for 1 hr. The DNA was phenol extracted and ethanol precipitated. The mixture of DNA molecules with plasmid origin of replication and ampicillin resistance gene all contain GAC at one end generated by the HincII cleavage and a variable sequence at the other end. Some molecules however were expected to have GTC between the TK gene transcriptional and translational starts at the opposing terminus. Upon ligation, these molecules would have a new SalI site (GTCGAC) generated. The mixture of molecules was ligated with 2 units of T4 DNA ligase (Boehringer Mannheim) at room temperature overnight in 20 mM Tris-HCl (pH 7.6), 10 mM DTT, 10 mM MgCl₂, 0.5 mM ATP. One µg of the ligated mixture was used to transform 100 µl of competent E. coli strain JM103 which were plated out on L-broth plates containing 50 µg/ml of ampicillin and grown at 37°C overnight. 144 single colonies were transferred to a master agar plate containing ampiciliin. The 144 colonies were arranged in 12 groups and each group was used to inoculate L-broth, ampicillin cultures. After overnight growth, minipreparations of plasmid were prepared and 2 µg of the purified DNAs were cleaved with 2 units of HindIII and 2 units of SalI for 2 hr at 37°C in 10 mM Tris-HCl (pH 7.5), 10 mM MgCl₂, 100 mM NaCl. One DNA preparation contained a fragment of approximately 500 bp which would be the size of a fragment produced by HindIII and SalI if a SalI site had been generated after the TK transcriptional start site. The 12 colonies used to make this DNA preparation were grown singly in L-broth cultures and plasmid DNA purified and cleaved with HindIII and SalI. From one of the plasmids, a fragment of approximately 500 bp was detected by agarose gel electrophoresis. This fragment was recloned in phage M13mp8 and M13mp9 and the nucleotide sequence was determined for 100 nucleotides from both the HindIII and SalI sites according to the Sanger dideoxy chain termination method to prove that it had the desired sequences. The plasmid containing the HindIII- SalI fragment was designated pMM1 and was purified from a one liter culture of transformed E. coli.
(b) Construction of pMM2. 5 g of a pUC9 derivative containing the vaccinia virus DNA HindIII J fragment was cleaved with 5 units of XhoI for 2 hr at 37°C in 10 mM (Tris-HCl) pH 7.5, 10 mM MgCl₂, 150 mM NaCl (high salt restriction buffer). The buffer composition was adjusted to 40 mM dATP, dCTP, dGTP and dTTP, 250 mM NaCl, 75 mM Tris-HCl (pH 8.8). One unit of E. coli DNA polymerase I (large fragment) was added and the reaction incubated at room temperature for 1 hr in order to give blunt ends. Synthetic EcoRI linkers were phosphorylated at their 5 termini by incubation with polynucleotide kinase as described previously. Phosphorylated linkers were ligated at room temperature to the blunt end DNA fragment as described previously. One mg of ligated mixture was used to transform E. coli JM103 and single ampicillin resistant colonies were picked and plasmids screened for a new EcoRI site where there had previously been an XhoI site. The plasmid with a new EcoRI site was designated pMM2.
(c) Construction of pMM3. By inserting the newly created EcoRI fragment that contains part of the TK gene from pMM2 into the EcoRI site of pMM1, a new plasmid was obtained that contains a number of restriction sites for insertion of a foreign gene coding sequence. 50 mg of pMM2 was cleaved with 75 units of EcoRI for 2 hr at 37°C in high salt restriction buffer and the 1 kb EcoRI fragment was purified by agarose gel electrophoresis and electroblotting onto DEAE paper. Five mg of pMM1 was cleaved with 5 units of EcoRI for 2 hr at 37°C as described previously. 1.5 units of bacterial alkaline phosphatase was added to the restriction digest and incubated for a further 30 min. The reaction mixture was then phenol extracted, chloroform extracted and ethanol precipitated. 0.25 mg of the EcoRI cleaved and alkaline phosphatase treated pMM1 was added to 0.5 mg of the isolated EcoRI fragment from pMM2 and the DNAs were ligated overnight. The ligated mixture was used to transform E. coli JM103 and plasmids were screened for a 1 kb EcoRI fragment inserted into pMM1 in the same orientation as present in the vaccinia genome. The resulting plasmid designated pMM3 contains unique HincII, AccI, SalI, BamHI and SmaI sites for insertion of foreign genes next to the thymidine kinase promoter.
(d) Construction of pMM4. In order to have an EcoRI site to insert foreign genes under control of the TK promoter, it was first necessary to remove the second EcoRI site distal to the TK promoter. This was achieved in the following manner. 100 mg of pMM2 was partially cleaved with 20 units of EcoRI for 15 min at 37°C in high salt restriction buffer and the linear DNA molecule formed by cleavage at a single EcoRI site was isolated by agarose gel electrophoresis and electroblotted onto DEAE paper. 250 mg of the linear DNA molecule was incubated with 0.5 units of E. coli DNA polymerase I (large fragment) at 15°C for 1 hr in 40 mM dATP, dCTP, dGTP, dTTP, 250 mM NaCl, 75 mM Tris-HCl (pH 8.8). The reaction mixture was then phenol extracted, chloroform extracted and ethanol precipitated. After ligation, the plasmid was used to transform E. coli JM103. The resulting transformed E. coli were screened for the presence of a plasmid with the EcoRI site farthest from the promoter deleted. The plasmid, designated pMM4, contains unique HincII, AccI, SalI, BamHI, SmaI, and EcoRI sites for insertion of foreign genes.

### Example 3 Formation of vaccinia virus recombinants that express the prokaryotic chloramphenicol acetyltransferase (CAT) gene.

(a) Insertion of the CAT gene into pGS21. A 770 bp DNA fragment containing the CAT gene was isolated from pBR328 by cleavage of pBR328 DNA with restriction endonuclease TagI followed by agarose gel electrophoresis, electroblotting onto DEAE-cellulose, elution and recovery of DNA by ethanol precipitation and centrifugation. This 770 bp DNA fragment was inserted into plasmid pUC7 as follows. pUC7 DNA was cleaved with restriction enzyme AccI, the 5' terminal phosphates were removed with calf intestinal alkaline phosphatase and the DNA was recovered after phenol:chloroform extraction by ethanol precipitation. 0.5 µg of linearized dephosphorylated plasmid DNA was ligated with 0.2 µg of the 770 bp fragment under standard conditions described above. Ligated DNA was then used to transform E. coli strain JM103 and white bacterial colonies that grew on 1.5% bacto-agar plates containing L-broth, 50 µg/ml of ampicillin, X-gal and IPTG, were picked and grown in L-broth. Mini-preparation of plasmid DNA were screened for the presence of the 770 bp DNA fragment containing the CAT gene by digestion with BamHi and agarose gel electrophoresis. Such a plasmid was grown, amplified and purified by standard procedures as described above and called pGS29.
   Plasmid pUC7 contains 2 BamHI sites closely flanking the AccI sites. Consequently, after insertion of the CAT gene into the AccI site, this gene was now excisable as a 780 bp fragment by BamHI. After BamHI digestion of pGS29, the 780 bp fragment was isolated by agarose gel electrophoresis, electroblotted onto DEAE-cellulose, eluted and recovered by ethanol precipitation.
   The next step was the insertion of this BamHI DNA fragment into plasmid vector pGS21. pGS21 DNA was linearized by cleavage with BamHI, the 5' terminal phosphates were removed by digestion with calf intestinal alkaline phosphatase and DNA was recovered by phenol:chloroform extraction and ethanol precipitation. 0.5 µg of linearized, dephosphorylated pGS21 DNA was ligated with 0.1 µg of the 780 bp DNA fragment under standard conditions and the ligated DNA was then used to transform, competent E. coli cells strain HB101. Transformed cells were plated onto an L-broth plate containing 1.5% bacto-agar and 50 g/ml ampicillin. After incubation for 15 hr at 37°C, bacterial colonies were picked grown in L-broth containing 50 µg/ml ampicillin and plasmid DNA was purified by the minipreparation procedure. Plasmid DNA was screened for the presence of the 780 bp CAT gene BamHI fragment in the correct orientation with respect to the vaccinia promoter by digestion with BamHI or EcoRI followed by agarose gel electrophoresis. Such a clone was called pGS24 and was grown, amplified and purified as described above.
(b) Insertion of the chimeric CAT gene into vaccinia virus. A 25 sq cm monolayer of TK⁻143 was infected with wild type vaccinia virus at 0.01 pfu/cell. A mixture of 1 µg of pGS24, 1 µg vaccinia virus DNA, and 20 µg of calf thymus DNA was precipitated with 125 mM CaCl₂. The fine suspension was used to transfect the cells at 2 hr after infection. After 30 min at 25°C, 7.2 ml of Eagle medium containing 8% fetal bovine serum was added and the monolayer was incubated for 3.5 hr at 37°C. The culture medium was then removed and replaced by 8 ml fresh Eagle medium containing 8% fetal bovine serum and the incubation was continued at 37°C for two days. Cells were scraped from the bottles, pelleted by centrifugation (2,000 X g, 5 min) and resuspended in 0.5 ml of Eagle medium containing 2.5% fetal bovine serum.
(c) Selection of recombinant vaccinia virus containing the chimeric CAT gene. Thymidine kinase negative vaccinia virus recombinants were selected by plaque assay in TK⁻143 cells with a 1% low melting agarose overlay containing 25 µg/ml BUdR. After three days at 37°C, cell monolayers were stained with 0.005% neutral red, plaques were picked using a sterile Pasteur pipette and placed in 0.5 ml of Eagle medium containing 2.5% fetal bovine serum. After freezing and thawing 3 times and sonication, 0.25 ml of each plaque was used to infect 16 mm diameter monolayers of TK⁻143 cells. Two hr after infection, culture medium was removed and monolayers were overlayed with 1 ml of eagle medium containing 2.5% fetal bovine serum and 25 g/ml BUdR. After two days incubation at 37°C, cell monolayers were scraped from wells, pelleted by centrifugation, resuspended in 0.5 ml of 0.15 M NaCl, 10 mM Tris-HCl (pH 7.5), frozen and thawed 3 times and 0.1 ml was transferred to nitrocellulose using a micro filtration manifold (Schleicher and Schuell). After procedures to denature, neutralize and fix DNA to the nitrocellulose, the filter was hybridized with ³²P-labeled CAT gene DNA. After washing the filter, an autoradiograph was obtained. Virus recombinants showing positive hybridization to ³²P-CAT DNA were further plaque purified in TK⁻143 cells with BUdR selection and the screening procedure for CAT DNA repeated. A clone positive for CAT DNA at this stage was then amplified once in TK⁻ cells with BUdR selection, once in CV-1 cells without selection and the virus titre determined by plaque assay in CV-1 cells. This virus was called vCAT24.
(d) Analysis of expression of chimeric CAT gene. CV-1 cells were infected with vCAT24 at 10 pfu/cell. After 24 hr at 37°C, cells were scraped, pelleted, resuspended in 0.2 ml of 0.25M Tris-HCl (pH 7.8) and sonicated. Cell debris was removed by centrifugation (12,000 X g, 5 min) and the supernatant was assayued for chloramphenicol acetyltransferase activity essentially as described by Gorman et al., Mol. Cell Biol. 2:1044-1051 (1982). Extracts from cells infected with vCAT24 contained an enzyme activity that acetylated chloramphenicol as demonstrated by thin layer chromatography. Extracts from both uninfected and wild-type vaccinia virus infected cells contained no detectable chloramphenicol acetyltransferase activity.

### Example 4 Construction of vaccinia virus expressing chimeric herpes thymidine kinase (HTK) gene.

50 µg of a plasmid containing a BamHI fragment including the HTK gene [Enguist et al., Gene 7:335-342 (1979)] was cleaved with 50 units of HincII and 50 units of PvuII for 2 hr at 37°C in medium salt restriction buffer. A 1.8 kb HincII-PVUII fragment devoid of the HTK transcriptional start site but containing all of its coding sequence was isolated by agarose gel electrophoresis and electroblotted onto DEAE paper. Five µg of pUC7 was cleaved with 5 units of HincII for 2 hr at 37°C in medium salt restriction buffer. 1.5 units of calf intestinal alkaline phosphatase was added and the reaction mixture was incubated for a further 30 min after which it was phenol extracted, chloroform extracted and ethanol precipitated. 250 ng of this DNA was ligated with 500 ng of the isolated HincII-PvuII HTK fragment and E. coli JM103 cells were transformed with the ligated mixture. Plasmids were isolated from the transformed bacteria and screened for the HincII-PvuII fragment inserted into pUC7. This plasmid was designated pVH4. 50 µg of pVH4 was cleaved with EcoRI and the EcoRI fragment containing the HTK gene was isolated by agarose gel electrophoresis and electroblotted onto DEAE paper. Five µg of pMM4 was cleaved with EcoRI; 1.5 units of calf intestinal alkaline phosphatase was added and the reaction incubated for a further 30 min. The reaction mixture was then phenol extracted, chloroform extracted and ethanol precipitated. 250 ng of this cleaved plasmid was ligated to 500 ng of the EcoRI fragment containing the HTK coding sequences and this ligated mixture was used to transform E. coli JM103. Single ampicillin resistant colonies were screened for a plasmid containing HTK coding sequences at the EcoRI site of pMM4. This recombinant plasmid was designated pMM20 and combines the vaccinia virus TK transcriptional regulatory sequence with an uninterrupted Herpes TK coding sequences, and the distal half of the vaccinia virus TK gene. This chimeric gene is flanked with vaccinia sequences such that homologous recombinantion would occur at the site of the vaccinia virus TK gene of wild type virus.

Human TK⁻143 cells were infected with 0.01 pfu/cell of a vaccinia virus TK⁻ mutant (TK⁻13; Bajszar et al., J. Virol., supra). The TK lesion maps between the vaccinia TK transcriptional start site and the EcoRI site in the vaccinia TK gene and hence the lesion could not be restored to wild type vaccinia TK by recombination with pMM20. One g of calcium phosphate precipitated pMM20 DNA was introduced by transfection into TK⁻143 cells infected with TK⁻13 vaccinia virus. Selection procedures described earlier for selection of TK⁺ virus were used. Isolated single TK⁺ plaques were plaque purified again and checked for synthesis of the herpesvirus TK. Since [I¹²⁵]dC is a specific substrate for the herpesvirus TK, but not for vaccinia virus or cellular TK, [I¹²⁵]dC is incorporated into viral DNA only if herpes virus TK is expressed. Autoradiography of cell monolayers, infected with the putative recombinant virus, in the presence of [I¹²⁵]dC revealed dark spots on the film corresponding to viral plaques showing that the TK⁺ virus was expressing herpesvirus TK. That the herpes TK was integrated into the viral genome was shown by DNA·DNA hybridization of ³²P-labeled HTK DNA to blots of separated restriction digests of purified recombinant viral DNA. A further confirmation that herpesvirus TK was expressed was obtained by plaquing a recombinant virus stock in the presence and absence of 40 µg/ml bromodeoxycytidine (BCdR). Wild type vaccinia virus plaques were slightly reduced in size but the number of plaques remained constant when the media was supplemented with 40 µg/ml BCdR. However, the titer of recombinant virus was reduced between 10 to 100-fold in the presence of 40 µg/ml BCdR, as expected if synthesis of the herpesvirus TK had occurred.

### Example 5 Construction of vaccinia virus recombinants expressing chimeric vesicular stomatitis virus (VSV) N gene.

50 µg of pJS223 [a plasmid containing a cDNA copy of VSV N gene, Sprague et al., J. Virol. 45:773 (1983)] was cleaved with 50 units of XhoI for 2 hr at 37°C in high salt restriction buffer. The smaller fragment containing N gene DNA was isolated by agarose gel electrophoresis and electroblotting onto DEAE paper. Five µg of pMM3 was cleaved with 5 units of SalI for 2 hr at 37°C. 1.5 units of calf intestinal alkaline phosphatase was added for a further 30 min. The reaction mixture was phenol extracted, chloroform extracted, and ethanol precipitated. 250 ng of this DNA as ligated to 500 ng of the isolated XhoI fragment and E. coli JM103 were transformed with the ligated mixture. Single ampicillin resistant colonies were screened for the presence of VSV cDNA cloned into pMM3. The chimeric plasmid containing the vaccinia promoter contiguous with the VSV cDNA was designated pMM17. Cells were infected with wild type vaccinia virus and transfected with this plasmid. After 48 hr, the cells were disrupted and TK⁻ virus was selected by plaque formation with BUdR in the agar overlay. Expression of the chimeric VSV N gene was shown by standard immunoprecipitation methods. Recombinant virus was used to infect cells at 20 pfu/cell and [³⁵S]methionine was added to the medium. At 6 hr after infection, a cytoplasmic extract was made from the infected cells. Rabbit anti-VSV antiserum and staph A protein were used to precipitate VSV proteins. After dissociation of the Staph A -protein complex, the proteins were analyzed on a 10% polyacrylamide gel in parallel with authentic VSV labeled proteins. After fluorography, a protein that was immunoprecipitated from cells infected with the recombinant virus was seen to comigrate with authentic VSV N protein. This result demonstrated that the vaccinia virus recombinant expressed the chimeric VSV N gene.

### Example 6 Construction of vaccinia virus expressing chimeric hepatis B virus surface antigen (HBsAg) gene.

(a) Insertion of the HBsAg gene in pGS20, pGS21, and pMM3. A 1,350 bp DNA fragment containing the HBsAg gene was obtained from a plasmid [Moriarity et al., Proc. Natl. Acad. Sci. USA 78:2606-2670 (1981)]. Nucleotide sequence data suggested that the first ATG codon after one of the BamHI restriction endonuclease sites represented the initial methionine residue of HBsAg. The fragment containing the HBsAg gene was isolated from 50 g of this plasmid by digestion with 100 units of BamHI in 50 mM NaCl, 10 mM Tris-HCl (pH 7.5), 10 mM MgSO₄, 10 mM dithiothrietol (DTT) (hereafter called medium restriction buffer) for 2 hr at 37°C. DNA fragments were separated by electrophoresis at 200 volts for 1 hr through a 1% agarose gel containing 40 mM Tris-acetate (Ac) (pH 8.0), 20 mM NaAc, 2 mM EDTA, 18 mM NaCl. The gel was soaked in 1 µg/ml ethidium bromide (EtBr) and DNA fragments were visualized by illumination with long wave ultraviolet light. A gel strip containing a DNA fragment of 1.35 kilobase pairs (kb) was excised from the agarose gel and DNA within this strip was transferred to diethylaminoethyl (DEAE)-cellulose by electroblotting in 40 mM Tris-Ac (pH 7.2), 20 mM NaAc, 1 mM EDTA for 1 hr at 2.5 mA. DNA was eluted from the DEAE-cellulose paper by shaking in 1.2 M NaCl, 40 mM Tris-Ac (pH 7.2), 20 mM NaAc, 1 mM EDTA for 30 min at 25°C and recovered from the supernatant by addition of 2 volumes of ethanol followed by centrifugation at 12,000 X g for 5 min.
   The plasmids pGS20, pGS21, and pMM3 were linearized by digestion with 2 units of BamHI/µg DNA in medium salt restriction buffer for 2 hr at 37°C. The linearized plasmids were then dephosphorylated at their 5' termini by incubation with 0.1 unit of calf intestinal alkaline phosphatase in 50 mM Tris-HCl (pH 9.0), 1 mM MgCl₂, 0.1 mM ZnCl₂, 1 mM spermidine for 30 min at 37°C. After two extractions with equal volumes of phenol:chloroform (1:1), the DNA was recovered by ethanol precipitation and centrifugation. 0.5 µg of each linearized, dephosphorylated plasmid was ligated with 0.2 µg of the 1.35 kb BamHI fragment containing the HBsAg gene in 66 mM Tris-HCl (pH 7.5), 6.6 mM MgCl₂, 10 mM DTT, 0.5 mM ATP for 15 hr at 12°C. Ligated DNA was used to transform competent E. coli cells strain HB101 and the transformed cells were grown for 15 hr at 37°C on L-broth plates containing 1.5% bacto-agar and 50 g/ml ampicillin. Bacterial colonies were picked and grown in 10 ml of L-broth containing 50 µg/ml of ampicillin for 15 hr at 37°C. Plasmid DNA was prepared from 1.5 ml of bacterial cultures by the following minipreparation of plasmid DNA. Bacterial cells were pelleted by centrifugation (12,000 X g, 1 min) and resuspended in 0.1 ml of lysis solution [25 mM Tris-HCl (pH 8.0), 10 mM EDTA, 50 mM glucose, 2 g/ml lysozyme] and incubated on ice for 30 min. 0.2 ml of 0.2 M NaOH, 1% sodium dodecyl sulfate (SDS) was added and the mixture incubated on ice for 5 min. 0.15 ml of 3M NaAc (pH 4.8) was added and after a further 1 hr incubation on ice, the mixture was centrifuged at 12,000 X g for 5 min. Plasmid DNA was precipitated from the supernatant by addition of 1 ml of ethanol, recovered by centrifugation (12,000 X g, 5 min) and finally redissolved in 0.1 ml of 10 mM Tris-HCl (pH 8.0) 1 mM EDTA (TE buffer).
   Plasmid DNAs were screened for the presence of the 1.35 kb BamHI fragment containing the HBsAg gene by digestion of 10% of each plasmid DNA sample with 5 units of restriction endonuclease BamHI in medium salt restriction buffer for 1 hr at 37°C, followed by agarose gel electrophoresis and EtBr staining.
   Since the 1.35 kb BamHI hepatitis B virus DNA fragment could be inserted in either of two orientations within each of the plasmids, additional screening was necessary. Plasmids derived from pGS20 and pGS21 were digested with XbaI restriction endonuclease in 100 mM NaCl, 50 mM Tris-HCl (pH 7.5), 10 mM MgSO₄ (high salt restriction buffer) for 1 hr at 37°C and analyzed by agarose gel electrophoresis. The identification of XbaI fragments of approximately 830 or 1,730 bp discriminated between derivatives of pGS20 that contained the HBsAg gene in incorrect and correct orientations, respectively. XbaI fragments of 2,150 bp or 1,150 bp discriminated between derivatives of pGS21 that had the HBsAg gene in incorrect and correct orientations, respectively. Plasmids derived from pMM3 were screened by HincII digestion in medium salt restriction buffer followed by agarose gel electrophoresis. The presence of the HBsAg gene in correct orientation was indicated by generation of fragments of approximately 5,400 bp and 200 bp whereas fragments of 4,400 bp and 1,200 bp resulted when HBsAg was in the incorrect orientation. Plasmids were then grown, amplified and purified as follows. Transformed bacteria were seeded into 400 ml cultures of M-9 medium containing 150 µg/ml leucine, 150 µg/ml proline, 0.8 µg/ml vitamin B₁, 50 µg/ml ampicillin and grown at 37°C until the culture reached an optical density of 0.8 at 590 nm. Chloramphenicol was then added to a concentration of 200 µg/ml and the culture was incubated for 12 hr at 37°C. Bacteria were pelleted by centrifugation (5,000 X g, 10 min), washed once in 0.15 M NaCl, 10 mM Tris-HCl (pH 7.5) and then resuspended in 10 ml of lysis solution (above). After incubation for 30 min on ice, 20 ml of 0.2 M NaOH, 1% SDS was added and incubation continued for 5 min. 15 ml of 3 M NaAc (pH 4.8) was then added and after incubation on ice for a further 1 hr, the mixture was centrifuged at 10,000 X g for 10 min. The supernatant was recentrifuged at 10,000 X g for 10 min and plasmid DNA was precipitated from the final supernatant by addition of 2 volumes of ethanol. After centrifugation at 10,000 X g for 5 min, the pellet was redissolved in 10 ml of TE buffer and the solution was extracted twice with equal volumes of phenol:chloroform. DNA was recovered by ethanol precipitation and centrifugation and redissolved in 5 ml of TE buffer. 0.1 mg/ml of ribonuclease (heated at 100°C to inactivate contaminating deoxyribonucleases) was added and the mixture was incubated for 30 min at 37°C DNA was then precipitated by addition of NaAc (pH 7) to 0.1 M and 1.5 volumes of ethanol and was recovered by centrifugation at 5,000 X g for 5 min. Remaining RNA was removed from the DNA by dissolving the pellet in 0.3 M NaCl, 10 mM Tris-HCl (pH 7.5), 10 mM EDTA and passage through a sephacryl-S300 column equilibrated with the same buffer. DNA eluting in the first peak was recovered by ethanol precipitation and centrifugation, dissolved in 1.0 ml of TE buffer and stored at 4°C. Plasmids from pGS21 that have the HBsAg gene in incorrect and correct orientations relative to the translocated promoter have been designated pHBs1 and pHBs2, respectively. Plasmids from pGS20 that have the HBsAg gene in incorrect and correct orientations have been designated pHBs3 and pHBs4. The plasmid from pMM3 that has the HBsAg gene in correct orientation relative to the TK promoter has been designated pHBs5.
(b) Formation of vaccinia virus recombinants containing a chimeric HBsAg gene. Plasmids containing a chimeric HBsAg gene flanked by segments of the vaccinia virus TK gene were used to transfect cells infected with wild-type thymidine kinase positive (TK⁺) vaccinia virus. Confluent monolayers of CV-1 or MRC-5 cells (25 sq cm) were infected with vaccinia virus at 0.01 plaque forming units (pfu) per cell. One µg of plasmid, 1 µg of vaccinia virus DNA and 20 µg of calf thymus DNA were mixed in 1 ml of 0.1% dextrose, 0.14 M NaCl, 5 mM KCl, 1 mM Na₂HPO₄, 20 mM Hepes and precipitated by addition of CaCl₂ to a final concentration of 0.125 M. The mixture was agitated gently for 45 min at 25°C and 0.8 ml of the fine suspension was added at 2 hr after infection to a monolayer from which medium had been removed. After 30 min at 25°C, 7.2 ml of Eagle medium containing 8% fetal bovine serum (FBS) was added and the incubation was continued at 37°C for a further 3.5 hr. At 6 hr after infection, the medium was replaced with fresh medium containing 8% FBS and the incubation was continued for 2 days. Cells were then scraped from the bottle, pelleted, resuspended in 0.5 ml of tissue culture medium and homogenized to break cells and liberate the virus.
   Only a small percentage of the virus produced in transfected cells was recombinant. These recombinants were isolated by selective and non-selective methods. Selection was possible because the HBsAg coding sequences were inserted into the TK gene and interrupted its function. Because of the thymidine kinase negative (TK⁻) phenotype, recombinant virus was able to form plaques in the presence of 5-bromodeoxyuridine (BUdR) whereas the original TK⁺ virus did not. Non-selective procedures were carried out at described by Villarreal and Berg [Science 196:181-185 (1977)].
   Monolavers of TK⁻143 cells were inoculated with virus from transfected cells and 2 hr later were overlayed with medium containing 1% low melting agarose, 25 µg/ml BUdR, and 2.5% FBS. After 3 days at 37°C in a humidified 5% CO₂ atmosphere, the cells were stained with 0.005% neutral red. Greater than 50% of the plaques visualized under these conditions were shown by dot blot hybridization to contain recombinants with hepatitis B virus DNA. For the latter procedure, individual virus plaques were picked using a sterile Pasteur pipette and used to infect TK⁻143 cell monolayers in 16 mm diameter multiwell dishes. After 48 hr of incubation in medium containing BUdR at 37°C, the cells were scraped, lysed by three Freeze-thaw cycles, and collected on nitrocellulose sheets by filtration through a micro-sample manifold (Schleicher and Schuell, NH). The filter was washed with 100 mM NaCl, 50 mM Tris-HCl, pH 7.5), blotted 3 times on successive Whatman 3 MM filter papers saturated with (1) 0.5 M NaOH, (2) 1 M Tris-HCl (pH 7.5), and (3) 2 X SSC (SSC is 0.15 M NaCl, 0.015 M sodium citrate), baked at 80°C for 2 hr and then incubated with 5 X Denhardt's solution [Denhardt, Biochem. Biophys. Res. Commun. 23:641-646 (1966)] supplemented with 0.1 mg/ml of denatured, sheared salmon sperm DNA in 4 X SSC at 65°C for 4 hr. Hepatitis B virus DNA, labeled with ³²P by nick translation [Rigby et al., J. Mol. Biol. 113, 237 (1975)], and sodium dodecyl sulfate (SDS) at a final concentration of 0.1% were added and hybridization continued for 15 hr at 65°C. The filter was washed twice for 15 min at 65°C with 2 X SSC/0.1% SDS and then with 0.2 X SSC/0.1% SDS. An autoradiograph was made by placing the filter next to X-ray film and the presence of dark spots on developed film identified recombinant virus containing the HBsAg gene. Recombinant virus was plaque purified a second time in TK⁻143 cells with an agar overlay containing 25 µg/ml of BUdR and recombinant virus stocks were then prepared. The recombinant virus stocks were designated vHBs1, vHBs2, vHBs3, vHBs4 and vHBs5 to correspond with the names given to the plasmids from which they were derived.
(c) Expression of HBsAg in cells infected with vaccinia virus recombinants. Evidence for HBsAg expression in cells infected with vaccinia virus recombinants was obtained by a standard radioimmunoassay procedure (ASURIA, Abbot Laboratories). Monolayers of CV-1 cells were infected with 10 plaque forming units (pfu) per cell of vaccinia virus recombinants, wild-type vaccinia virus, or mock infected as indicated in Table 1. Two hr after infection, the virus inoculum was removed and cells were overlayed with Eagles medium containing 2.5% FBS. After incubation for 24 hr, the cells were scraped from culture flasks and separated from culture medium by centrifugation (2,000 X g, 5 min). Cell pellets were resuspended in 0.5 ml of TBS [0.15 M NaCl, 10 mM Tris-HCl (pH 7.5)], frozen and thawed 3 times, sonicated and centrifuged (12,000 X g, 1 min) to remove debris. The supernatant and tissue culture medium were assayed for HBsAg by radioimmunoassay. Beads coated with anti-HBsAg antibody were incubated with 0.2 ml of sample for 2 hr at 45°C, thoroughly rinsed 8 times with 1 ml H₂O and then reincubated with ¹²⁵I-labeled antibody against HBsAg for 1 hr at 45°C. Beads were washed again and then counted in a gamma-ray scintillation counter. The quantity of HBsAg present was calculated by reference to positive and negative controls supplied by Abbot Laboratories and incubated in parallel.
   As shown in Table 1, HBsAg was produced in greatest amount by cells infected with vHBs2 and vHBs4. Lesser amounts of HBsAg were made in cells infected with vHBs5. By contrast, those recombinants that did not have the HBsAg gene in correct orientation with the translocated vaccinia virus promoter, vHBs1 and vHBs3, made barely detectable levels of HBsAg. Significantly, much of the HBsAg was excreted into the culture medium. Release of HBsAg was not due to cell lysis since 90% of infectious virus remained cell associated. Table 1 also shows that the similar yields of virus were obtained after infection with wild-type (WT) or recombinant virus.
   The nature of the HBsAg synthesized by cells infected with vaccinia virus recombinants was analyzed by immunoprecipitation. Monolayers or CV-1 cells infected with purified vaccinia virus recombinants at 30 pfu/cell were incubated in Eagles medium containing 0.01 mM methionine for 4 hr after infection. The cells were then incubated for 20 min at 37°C with fresh Eagles medium without methionine supplemented with [³⁵S]methionine (120 µCi/5 X 10⁶ cells). Excess [³⁵S]methionine was removed bv washing the cells 3 times with ice-cold phosphate buffered saline and cell extracts were prepared by incubation of cells in 0.5 ml of 0.1% aprotinin, 0.1 M Tris-HCl (pH 8.0), 0.1 M NaCl, 0.5% NP40, for 10 min on ice, followed by centrifugation. 80% of the supernatant was incubated with 25 µl of guinea pig non-immune serum at 4°C for 15 hr and immune complexes were removed by addition of 50 µl of a formalin-treated staphylococcal A suspension and incubation for 30 min at 4°C, followed by centrifugation. 20 µl of guinea pig HBsAg antiserum was then added, incubated for 15 hr at 4°C and then immune complexes were removed by addition of staphylococcal A suspension as above, followed by centrifugation. The pellet was washed twice in 0.05 M Tris-HCl (pH 7.5), 0.15 M NaCl, 0.1% SDS, 1% Triton X-100, 1% sodium deoxycholate and twice in 0.4M LiCl, 2M urea, 10 mM Tris-HCl (pH 8.0). Immune complexes were eluted from the staphylococcal A pellet by incubation in 50, µl of 0.06 M Tris-HCl (pH 6.8), 3% SDS, 5% β-mercaptoethanol, 10% glycerol, 0.002% bromophenol blue for 15 min at 25°C. After centrifugation, the supernatant was boiled and electrophoresed through a 15% polyacrylamide gel. The gel was fixed, treated with Enhance (New England Nuclear Corporation) and a fluorograph was obtained. Examination of the fluorograph indicated that two polypeptides were specifically immunoprecipitated. These had molecular weights of 23,000 and 25,400 and comigrated on polyacrylamide gels with polypeptides immunoprecipitated by HBsAg antiserum from a hepatoma cell line PLC/PRF/5. The nature of the HBsAg excreted from cells infected with vaccinia virus recombinants was further examined. Tissue culture medium, harvested 24 hr after infection of CV-1 cells with 30 pfu/cell of vaccinia virus recombinant vHBs4, was clarified by centrifugation at 2,000 X g for 5 min and then recentrifuged at 75,000 X g for 24 hr at 4°C. The pellet was resuspended in 4.5 ml of 10 mM Tris-HCl (pH 7.5), 150 mM NaCl, 1 mM EDTA and then 1.38 grams of CsCl was added to a final density or 1.2 g/cm³. The sample was centrifuged at 220,000 X g for 64 hr at 4°C and gradient fractions were collected. Diluted samples were tested for HBsAg by radioimmunoassay. A peak of HBsAg was detected at a density of 1.2 g/cm³ which was identical to a peak of HBsAg obtained when culture medium from hepatoma cell line PLC/PRF/5 was treated in parallel. The peak fractions of HBsAg were dialyzed against phosphate buffered saline (PBS) and recentrifuged on a 5 to 30% (w/w) sucrose gradient in PBS for 4.5 hr at 150,000 X g at 4°C. Gradient fractions were collected and samples were diluted and assayed for HBsAg by radioimmunoassay. A peak of HBsAg was detected which sedimented at the same rate as the HBsAg from hepatoma cell line PLC/PRF/5. When samples from these peaks were analyzed by electron microscopy, particles of HBsAg wre detected.
   In all respects examined (including antigenicity, polypeptide composition, buoyant density, sedimentation rate), the HBsAg excreted from cells infected with vaccinia virus recombinant vHBs4 was indistinguishable from HBsAg particles released from hepatoma cell line PLC/PRF/5.
(d) Vaccination of rabbits with vaccinia virus recombinants. Previous studies have shown that HBsAg particles from the blood of human hepatitis B virus carriers are highly immunogenic and can neutralize the infectivity of hepatitis B virus. Consequently, the question of whether infection of animals with vaccinia virus recombinants that express HBsAg would induce production of anti-HBVsAg antibodies was examined.
   Rabbits were pre-bled and then infected with either wild-type vaccinia virus or vaccinia virus recombinant vHBs4 by intradermal injection of 10⁸ pfu of virus into 4 sites on the back of each rabbit. Rabbits were bled daily following inoculation and serum was prepared and stored frozen at -70°C. At 5 days, the rabbits developed lesions at the sites of inoculation and by 10 days these lesions were visibly healing. Serum from the rabbits was tested from HBsAg and antibodies against HBsAg by radioimmunoassay, and for vaccinia virus by plaque assay. No HBsAg or vaccinia virus was detectable in the serum. However, by 5 days after inoculation, antibodies against HBsAg were detected (Table 2).

**TABLE 1**

| Production of HBsAg and Vaccinia Virus from Infected Cells | | | | |
|---|---|---|---|---|
| | ng HBsAg/5 X 10⁶ cells | | Virus yield (pfu) | |
| | cell extract | culture medium | cell extract | culture medium |
| Uninfected | <1 | <1 | ND | ND |
| WT | <1 | <1 | 7.8 X 10⁸ | 7.7 X 10⁷ |
| vHBs1 | 11 | 20 | 8.3 X 10⁸ | 10.2 X 10⁷ |
| vHBs2 | 835 | 1700 | 7.9 X 10⁸ | 9.9 X 10⁷ |
| vHBs3 | 14 | 25 | 9.1 X 108 | 9.0 X 10⁷ |
| vHBs4 | 930 | 1700 | 8.8 X 10⁸ | 9.8 X 10⁷ |
| vHBs5 | 35 | 80 | 10.3 X 10⁸ | 9.6 X 10⁷ |
| Hepatoma cells | 340 | 900 | ND | ND |

CV-1 cell monolayers were infected with purified wild-type (WT) or vaccinia virus recombinants (vHBs 1-5) at 30 plaque forming units (pfu)/cell or mock infected. At 2 hr, virus inoculum was replaced with 2.5 ml of Eagle medium containing 2.5% fetal bovine serum. Cells were harvested at 24 hr and separated from culture medium by centrifugation at 2,000 X g for 5 min. Cell pellets wee suspended in 2.5 ml of phosphate buffered saline, frozen and thawed 3 times and sonicated. Equal portions of cell extracts and culture medium were tested for HBsAg by radioimmunoassay and for vaccinia virus by plaque assay in CV-1 cells. Culture medium and a cell extract prepared as above from a hepatoma cell line PLC/PRF/5 three days after confluency were tested in parallel for HBsAg.

**TABLE 2**

| Production of antibodies against HBsAg by rabbits vacciniated with recombinant vHBs4 | | |
|---|---|---|
| Days after vaccination | RIA units/0.2 ml of serum | |
| | vHBs4 | WT virus |
| 5 | 92 | 8 |
| 6 | 135 | - |
| 7 | 352 | - |
| 8 | >512 | - |
| 9 | >512 | - |
| 10 | >512 | - |
| 11 | 442 | 8 |

Undiluted serum, obtained from rabbits on the days indicated, was tested for antibody to HBsAg by a radioimmunoassay procedure (AUSAB, Abbot Laboratories). An HBsAg positive control human plasma supplied by Abbot Laboratories had a titer of 512 RIA units.

### Deposition of Materials

A Sample of E. coli HB101 transformed with pGS20 has been deposited on November 30, 1982, at the American Type Culture Collection (ATCC), 12301 Parklawn Drive, Rockville, Maryland 20852 and has been given ATCC No. 39249. On December 1, 1982, samples of E. coli HB101 transformed with pHBs4 were deposited and assigned ATCC No. 39250, as were samples of vHBs4, which were assigned ATCC No. VR2055.

## Claims

1. A vector which can undergo homologous recombination in a poxvirus, comprising:
a chimeric gene which comprises at least one poxvirus transcriptional regulatory sequence and, under the transcriptional control of the regulatory sequence, at least one uninterrupted protein coding sequence from a foreign gene, wherein the regulatory sequence and the coding sequence are not separated by another transcriptional regulatory sequence; and
DNA, flanking the chimeric gene, from a non-essential region of poxvirus genome.

2. A vector according to claim 1, wherein the foreign gene is of prokaryotic chloramphenicol acetyltransferase, herpes thymidine kinase, vesicular stomatitis virus N, hepatitis B virus surface antigen, hepatitis B virus, hepatitis A virus, hepatitis non-A, non-B virus, influenza virus, cytomegalovirus, an adenovirus, a parvovirus, foot and mouth disease virus, poliovirus, measles virus, rabies virus, a coronavirus, a coxsackievirus, a pathogenic bacterium, rickettsia, a protazoon or a metazoon.

3. A vector according to claim 1, wherein the protein coding sequence encodes at least the antigenic portion of an immunogenic protein.

4. A vector according to claim 1, wherein the protein coding sequence encodes an immunogenic protein.

5. A vector according to any preceding claim, wherein the flanking DNA comprises a segment of the vaccinia virus thymidine kinase gene and DNA adjacent to said thymidine kinase gene.

6. A vector according to claim 5, wherein the flanking DNA comprises the vaccinia virus thymidine kinase gene.

7. A vector according to any preceding claim, wherein the transcriptional regulatory sequence regulates the vaccinia virus thymidine kinase gene or an early vaccinia virus gene encoding the 7.5 K polypeptide.

8. A vector according to any preceding claim, wherein the poxvirus DNA is vaccinia virus DNA.

9. A vector according to any preceding claim, wherein the protein coding sequence from a foreign gene comprises the site corresponding to initiation of translation of the foreign gene and DNA extending beyond the translational termination site of the foreign gene.

10. A vector according to claim 9, wherein the protein coding sequence is obtained from a DNA copy of a DNA gene or a DNA copy of a RNA gene.

11. An infectious poxvirus containing therein:
a chimeric gene as defined in any of claims 1 to 4 and 7 to 10; and
flanking DNA as defined in any of claims 1, 5 and 6,
such that the infectious poxvirus is capable, upon infection of a cell, of expressing the protein coding sequence in the chimeric gene.

12. An infectious poxvirus according to claim 11, wherein the chimeric gene comprises poxvirus DNA preceding and including the site at which RNA synthesis starts.

13. An infectious poxvirus according to claim 12, wherein the poxvirus DNA comprises a DNA segment containing about 275 or fewer base-pairs preceding the site at which RNA synthesis starts.

14. An infectious poxvirus according to any of claims 11 to 13, wherein the protein coding sequence encodes at least the antigenic portion of the hepatitis B virus surface antigen.

15. A method for preparing a vector according to any of claims 1 to 10, comprising the steps of:
(a) preparing a plasmid, cosmid or phage containing the flanking DNA flanking a poxvirus DNA transcriptional regulatory sequence including, adjacent to at least one restriction endonuclease site, the site at which RNA synthesis starts; and
(b) inserting the at least one uninterrupted protein coding sequence from a foreign gene into the restriction endonuclease site.

16. A method for preparing a recombinant infectious poxvirus according to any of claims 11 to 14, comprising the steps of:
(a) transfecting a cell infected with a genus of poxvirus with a vector according to any of claims 1 to 10 or obtainable by a method according to claim 15, wherein homologous recombination occurs between the DNA of the infecting poxvirus and at least a portion of the poxvirus DNA in the vector; and
(b) isolating from the cell the recombinant poxvirus.

## Patentansprüche

1. Vektor, der zur homologen Rekombination in ein Pockenvirus in der Lage ist, umfassend:
ein chimäres Gen, das wenigstens eine Regulationssequenz für die Transkription von Pockenvirus umfaßt, und, unter der Transkriptionskontrolle der Regulationssequenz, wenigstens eine ununterbrochene proteincodierende Sequenz eines fremden Gens, worin die Regulationssequenz und die codierende Sequenz nicht durch eine weitere Regulationssequenz für die Transkription getrennt sind; und
DNA aus einem nichtessentiellen Bereich des Pockenvirus-Genoms, die das chimäre Gen flankiert.

2. Vektor nach Anspruch 1, worin das fremde Gen von prokaryotischer Chloramphenicol-Acetyltransferase, Herpes Thymidin-Kinase, Vesicular Stomatitis-Virus N-Gen, Hepatitis-Virus Oberflächenantigen, Hepatitis B-Virus, Hepatitis A-Virus, Hepatitis non-A, non-B-Virus, Influenza-Virus, Cytomegalo-Virus, einem Adenovirus, einem Parvo-Virus, Maul- und Klauensäuche-Virus, Poliovirus, Masern-Virus, Tollwut-Virus, einem Corona-Virus, Coxsackie-Virus, einem pathogenen Bakterium, Rickettsien, einem Protozoon oder einem Metazoon stammt.

3. Vektor nach Anspruch 1, worin die proteincodierende Sequenz mindestens für den antigenen Teil eines immunogenen Proteins codiert.

4. Vektor nach Anspruch 1, worin die proteincodierende Sequenz für ein immunogenes Gen codiert.

5. Vektor nach irgendeinem der vorhergehenden Ansprüche, worin die flankierende DNA ein Segment des Thymidin-Kinase-Gens von Vaccinia-Virus und zu diesem Thymidin-Kinase-Gen benachbarte DNA umfaßt.

6. Vektor nach Anspruch 5, worin die flankierende DNA das Thymidin-Kinase-Gen von Vaccinia-Virus umfaßt.

7. Vektor nach irgendeinem der vorhergehenden Ansprüche, worin die Regulationssequenz für die Transkription das Thymidin-Kinase-Gen oder ein frühes Gen von Vaccinia-Virus, das für das 7,5 K Polypeptid codiert, kontrolliert.

8. Vektor nach irgendeinem vorhergehenden Anspruch, worin die Pockenvirus-DNA Vaccinia-Virus-DNA ist.

9. Vektor nach irgendeinem der vorhergehenden Ansprüche, worin die proteincodierende Sequenz eines fremden Gens die der Translationsinitiation entsprechende Sequenz des fremden Gens und DNA umfaßt, die jenseits der Terminationsstelle der Translation des fremden Gens liegt.

10. Vektor nach Anspruch 9, worin die proteincodierende Sequenz von einer DNA-Kopie eines DNA-Gens oder einer DNA-Kopie eines RNA-Gens erhalten ist.

11. Infektiöses Pockenvirus, das enthält:
ein chimäres Gen, wie es in irgendeinem der Ansprüche 1 bis 4 und 7 bis 10 definiert ist; und
flankierende DNA, wie sie in irgendeinem der Ansprüche 1, 5 und 6 definiert ist, so daß das Pockenvirus nach Infektion einer Zelle in der Lage ist, die proteincodierende Sequenz im chimären Gen zu exprimieren.

12. Infektiöses Pockenvirus nach Anspruch 11, worin das chimäre Gen Pockenvirus-DNA umfaßt, die der Sequenz, an der die RNA-Synthese beginnt, vorangeht und sie einschließt.

13. Infektiöses Pockenvirus nach Anspruch 12, worin die Pockenvirus-DNA ein DNA-Segment mit ungefähr 275 oder weniger Basenpaaren umfaßt, die der Stelle, an der die RNA-Synthese beginnt, vorangehen.

14. Infektiöses Pockenvirus nach irgendeinem der Ansprüche 11 bis 13, worin die proteincodierende Sequenz wenigstens für den antigenen Teil des Oberflächenantigens von Hepatitis B-Virus codiert.

15. Verfahren zur Herstellung eines Vektors nach irgendeinem der Ansprüche 1 bis 10, das die Schritte umfaßt:
(a) Herstellung eines Plasmids, Cosmids oder Phagen, die die flankierende DNA enthalten, die eine Regulationssequenz für die Transkription von Pockenvirus-DNA flankiert und, benachbart zu wenigstens einer Schnittstelle für eine Restriktionsendonuclease, die Stelle, an der die RNA-Synthese beginnt, einschließt; und
(b) Inserieren der für wenigstens ein ununterbrochenes Protein codierenden Sequenz eines fremden Gens in die Schnittstelle für die Restriktionsendonuclease.

16. Verfahren zur Herstellung eines rekombinanten infektiösen Pockenvirus nach irgendeinem der Ansprüche 11 bis 14, das die Schritte umfaßt:
(a) Transfektion einer mit einer Gattung eines Pockenvirus infizierten Zelle mit einem Vektor nach irgendeinem der Ansprüche 1 bis 10 oder erhältlich nach einem Verfahren nach Anspruch 15, in der eine homologe Rekombination zwischen der DNA des infizierenden Pockenvirus und wenigstens einem Teil der Pockenvirus-DNA im Vektor erfolgt; und
(b) Isolieren des rekombinanten Pockenvirus aus der Zelle.

## Revendications

1. Un vecteur qui peut subir une recombinaison homologue dans un poxvirus, comprenant:
un gène chimérique qui comprend au moins une séquence de régulation de la transcription du poxvirus et, sous le contrôle de la transcription de la séquence de régulation, au moins une séquence codant une protéine ininterrompue provenant d'un gène étranger, dans lequel la séquence de régulation et la séquence codante ne sont pas séparées par une autre séquence de régulation de la transcription; et
un ADN, flanquant le gène chimérique, provenant d'une région non-essentielle du génome d'un poxvirus.

2. Un vecteur selon la revendication 1, dans lequel le gène étranger est celui de la chloramphénicol acétyltransférase procaryote, de la thymidine kinase de l'herpès, du virus N de la stomatite vésiculaire, de l'antigène de surface du virus de l'hépatite B, du virus de l'hépatite B, du virus de l'hépatite A, de l'hépatite non-A, du virus non-B, du virus de la grippe, du cytomégalovirus, d'un adénovirus, d'un parvovirus, du virus de la fièvre aphteuse, du virus de la poliomyélite, du virus de la rougeole, du virus de la rage, d'un coronavirus, d'un virus Coxsackie, d'une bactérie pathogène, d'une rickettsie, d'un protozoaire ou d'un métazoaire.

3. Un vecteur selon la revendication 1, dans lequel la séquence codant la protéine code au moins la portion antigénique d'une protéine immunogène.

4. Un vecteur selon la revendication 1, dans lequel la séquence codant la protéine code une protéine immunogène.

5. Un vecteur selon l'une quelconque des revendications précédentes, dans lequel l'ADN placé sur les flancs comprend un segment du gène de la thymidine kinase du virus de la vaccine et l'ADN adjacent audit gène de la thymidine kinase.

6. Un vecteur selon la revendication 5, dans lequel l'ADN placé sur les flancs comprend le gène de la thymidine kinase du virus de la vaccine.

7. Un vecteur selon l'une quelconque des revendications précédentes, dans lequel la séquence de régulation de la transcription contrôle le gène de la thymidine kinase du virus de la vaccine ou un gène précoce du virus de la vaccine codant le polypeptide 7,5 K.

8. Un vecteur selon l'une quelconque des revendications précédentes, dans lequel l'ADN du poxvirus est l'ADN du virus de la vaccine.

9. Un vecteur selon l'une quelconque des revendications précédentes, dans lequel la séquence codant la protéine provenant d'un gène étranger comprend le site correspondant à l'initiation de la traduction du gène étranger et l'ADN s'étendant au delà du site de terminaison de la traduction du gène étranger.

10. Un vecteur selon la revendication 9, dans lequel la séquence codant la protéine est obtenue à partir d'une copie d'ADN d'un gène d'ADN ou d'une copie d'ADN d'un gène d'ARN.

11. Un poxvirus infectieux contenant à l'intérieur:
un gène chimérique comme défini dans l'une quelconque des revendications 1 à 4 et 7 à 10; et un ADN placé sur les flancs comme défini dans l'une quelconque des revendications 1, 5 et 6, de sorte que le poxvirus infectieux est capable, lors de l'infection d'une cellule, d'exprimer la séquence codant la protéine dans le gène chimérique.

12. Un poxvirus infectieux selon la revendication 11, dans lequel le gène chimérique comprend l'ADN du poxvirus précédant et incluant le site de départ de la synthèse de l'ARN.

13. Un poxvirus infectieux selon la revendication 12, dans lequel l'ADN du poxvirus comprend un segment d'ADN contenant environ 275 paires de bases ou moins, précédent le site de départ de la synthèse de l'ARN.

14. Un poxvirus infectieux selon l'une quelconque des revendications 11 à 13, dans lequel la séquence codant la protéine code au moins la portion antigénique de l'antigène de surface du virus de l'hépatite B.

15. Une méthode de préparation d'un vecteur selon l'une queconque des revendications 1 à 10, comprenant les étapes de:
(a) préparation d'un plasmide, d'un cosmide ou d'un phage contenant l'ADN placé sur les flancs, flanquant une séquence de régulation de la transcription de l'ADN d'un poxvirus comprenant, adjacent à au moins un site d'une endonucléase de restriction, le site de départ de la synthèse de l'ARN; et
(b) l'insertion d'au moins une séquence codant une protéine ininterrompue provenant d'un gène étranger dans le site de l'endonucléase de restriction.

16. Une méthode de préparation d'un poxvirus infectieux recombinant selon l'une quelconque des revendications 11 à 14 comprenant les étapes de:
(a) transfection d'une cellule infectée avec un genre de poxvirus avec un vecteur selon l'une queconque des revendications 1 à 10 ou que l'on peut obtenir par une méthode selon la revendication 15, dans laquelle une recombinaison homologue se produit entre l'ADN du poxvirus infectant et au moins une portion de l'ADN du poxvirus dans le vecteur; et
(b) isolement de la cellule du poxvirus recombinant.
